# EUROPÄISCHE PATENTSCHRIFT

(11) **EP 0 957 961 B1**
(45) Veröffentlichungstag und Bekanntmachung des Hinweises auf die Patenterteilung: **28.08.2002**
(21) Anmeldenummer: 96932365.8
(22) Anmeldetag: 02.10.1996
(51) Int. Cl.: A61M 15/00

(54) **INHALATIONSHILFSGERÄT**
INHALATION AID DEVICE
DISPOSITIF INHALATEUR AUXILIAIRE

(30) Priorität: 02.10.1995 AT 52695 U
(43) Veröffentlichungstag der Anmeldung: 24.11.1999
(73) Patentinhaber: WMC Mediation & Consulting Gmbh, 5020 Salzburg (AT)
(72) Erfinder: KLEIN, Elena, A-5084 Grossgmain-Hinterreit (AT)
(74) Vertreter: Miksovsky, Alexander, Dipl.-Ing.
(86) Internationale Anmeldenummer: AT9600177
(87) Internationale Veröffentlichungsnummer: WO97012638

(56) Entgegenhaltungen:
- EP-A- 0 384 050
- EP-A- 0 385 212
- GB-A- 2 110 543
- GB-A- 2 182 249
- GB-A- 2 248 400
- US-A- 4 484 577
- US-A- 5 318 016

## Beschreibung

Die vorliegende Erfindung bezieht sich auf ein Inhalationshilfsgerät zur Verwendung in Verbindung mit Aerosolabgabegeräten, umfassend eine Kammer, welche mit einer Öffnung für die Verbindung mit einem Aerosolabgabegerät und mit einer Öffnung mit einem Mundstück zum Abziehen des Aerosols ausgebildet ist, wobei die Kammer mit veränderbarem Volumen ausgebildet ist und wobei die Öffnung für die Verbindung mit dem Aerosolabgabegerät mit veränderbarem Querschnitt und/oder in einem Bodenelement der Kammer aus elastischem Material ausgebildet ist.

Dosier-Aerosole sind die überwiegend eingesetzten Verabreichungsformen von Medikamenten zur Behandlung von Asthma bronchiale und chronischer Bronchitis. Auch bei Allergien kommen die Dosier-Aerosole immer mehr zur Anwendung. Die inhalative Behandlung dieser Krankheiten mit Dosier-Aerosolen hat sich hiebei besonders bei akut auftretenden Beschwerden, wie Atemnot, als sehr wirkungsvoll erwiesen. Ein Dosier-Aerosol ist in der Regel in einem Medikamentenbehälter enthalten, welcher die einzelnen Komponenten, nämlich den mikronisierten Wirkstoff, Suspendierungsmittel und Treibgas beinhaltet. Durch Schütteln vor der Anwendung werden die einzelnen Komponenten vermischt. Nach jedem Sprühstoß werden diese Komponenten, wenn der Medikamentenbehälter senkrecht gehalten wird, der Dosierkammer des Medikamentenbehälters in genormter Menge neuerlich zugeführt und nur die in der Dosierkammer enthaltene Menge kann bei einem Sprühstoß ausgebracht werden.

Um die treibgashaltigen Dosier-Aerosole inhalieren zu können, werden Aerosolabgabegeräte, welche überwiegend L-förmig ausgebildet sind, wie dies beispielsweise der US-PS 3 994 421, der US-PS 4 509 515, der DE-OS 32 29 702 oder der DE-OS 38 16 276 zu entnehmen ist, eingesetzt. Die L-förmigen Aerosolabgabegeräte haben den großen Nachteil, daß bei diesen das Aerosol unkontrolliert und mit sehr hoher Geschwindigkeit aus dem Mundstück des Abgabegerätes austritt und so ein großer Teil des Aerosols, meist mehr als 80 %, bei jedem Sprühstoß im Mund-, Rachen- und Gaumenbereich kleben bleibt. Nur ca. 6 bis 20 % des Aerosols gelangen in den Respirationstrakt, wo sie ihren therapeutischen Effekt entwickeln können. Der hohe, im Mund-, Rachen- und Gaumenbereich verbleibende Medikamentenanteil kann hiebei bei diversen Dosier-Aerosolen unangenehme Nebenwirkungen verursachen, die gegebenenfalls wiederum mit zusätzlichen Medikamenten behandelt werden müssen. Bei derartigen Aerosolabgabegeräten dient somit der Mund-, Rachen- und Gaumenbereich als Zerstäubungskammer.

Alle Dosier-Aerosole sind im Funktionsaufbau im wesentlichen gleich. Die üblicherweise in dem Medikamentenbehälter enthaltene Suspension enthält mikronisierte Wirkstoffpartikel. Bei den üblichen L-förmigen Aerosolabgabegeräte sind die mikronisierten Wirkstoffpartikel beim Austritt aus der Düse mit sogenannten Primärtröpfchen ummantelt. Die Primärtröpfchen sind beim Düsenaustritt normalerweise so groß, daß sie nicht schwebfähig sind und wirken daher in dem Mund-, Rachen- und Gaumenbereich wie Geschosse. Darum werden aufbauend auf den L-förmigen Aerosolabgabegeräten weitere spezielle Inhalationshilfsgeräte, sogenannte Spacer, angeboten, die zu einer Verminderung der Medikamentenablagerungen im Mund-, Rachen- und Gaumenbereich führen sollen, wie dies beispielsweise aus der EP-A 0 009 667 bekannt geworden ist. Eine weitere Ausbildung eines Inhalationshilfsgerätes ist auch der EP-A 0 384 050 zu entnehmen, wobei ein in seinem Volumen nicht veränderbarer Zylinder an seinem Eintrittsende mit veränderbarem Querschnitt und an seinem Austrittsende mit einem Ventil ausgebildet ist.

Diese Inhalationshilfsgeräte sind großvolumige Zerstäubungskammern, in welche das Aerosol zuerst mit den L-förmigen Aerosolabgabegeräten hineingesprüht werden soll. Die beim Sprühstoß gebildeten Primärtröpfchen haben somit eine ausreichende Strecke zur Verfügung, um ihre hohe Geschwindigkeit abzubremsen. Durch das große Volumen des Spacers werden die Primärtröpfchen darüberhinaus angewärmt und können somit verdampfen.

Nach dem Verdampfen sollen hiebei möglichst viele Wirkstoffpartikel, die kleiner als 5 µm und somit lungengängig sind, im Spacer schweben wobei alle nicht schwebfähigen Medikamentenpartikel sich an der Wand des Spacers anlagern und dort verbleiben.

Wird bei der Anwendung von Dosier-Aerosolen mit üblichen L-förmigen Aerosolabgabegeräten kein derartiges zusätzliches Inhalationshilfsgerät verwendet, so schießen die Primärtröpfchen aufgrund ihrer Größe wie Geschosse in den Mundraum und bleiben, wie schon beschrieben, zum größten Teil an der Rachenwand kleben, weil keine ausreichende Strecke und kein ausreichendes Volumen vorhanden ist, damit die Primärtröpfchen verdampfen und ihre hohe Geschwindigkeit reduzieren können.

Bei der Anwendung eines Spacers in Kombination mit einem Aerosolabgabegerät verlagert sich dagegen die bisherige Mund-, Rachen- und Gaumendeposition des Dosier-Aerosols in den Spacer und somit kann die Gefahr von Nebenwirkungen, wie Mundsoor oder Entzündungen, nahezu gebannt bzw. weitestgehend verringert werden. Die Medikamentendeposition in der Lunge erhöht sich nur minimal. Ein weiterer Vorteil des Spacers besteht darin, daß aufgrund des großen Volumens die schwerbfähigen Wirkstoffpartikel einige Zeit im Spacer verbleiben und der Anwender genügend Zeit hat, das Aerosol aus dem Spacer zu inhalieren. Somit konnte weiters das Koordinationsproblem eines gleichzeitigen Auslösens eines Sprühstoßes des Aerosols und des Inhalierens gelöst werden. Der Spacer ist ein sinnvolles Produkt, um die Therapie mit den Dosier-Aerosolen zu verbessern, die subjektiven Nebenwirkungen zu verringern und das Koordinationsproblem zu lösen.

Das größte Problem bei der Nutzung eines derartigen Inhalationshilfsgerätes bzw. Spacers besteht allerdings in seinen großdimensionierten Abmessungen und derartige Spacer sind daher aufgrund ihrer Größe als Taschengeräte ungeeigenet und deshalb auch unpraktisch zu verwenden. Somit wird es für den Anwender fast unmöglich, den Spacer immer mit sich zu führen. Die Anwender von Dosier-Aerosolen müssen in der Regel mehrmals täglich ihre Dosier-Aerosole benutzen und würden hiebei gerne auf den Spacer als sinnvolles Hilfsmittel zurückgreifen.

Probleme mit großvolumigen Inhalationshilfsgeräten werden teilweise dadurch gemindert, daß derartige Spacer mit veränderbarem Volumen vorgeschlagen werden, wie dies beispielsweise der GB-A 2 110 543, der GB-A 2 182 249 oder der US-A 5 074 294 zu entnehmen ist. Bei diesen bekannten Inhalationshilfsgräten ist jedoch nachteilig, daß sie sehr komplizierte technische Konstruktionen darstellen, welche einen übermäßig großen konstruktiven Aufwand erfordern und somit nicht kostengünstig herstellbar sind.

Eine abgewandelte Ausführungsform eines Inhalationshilfsgerätes mit veränderbarem Volumen der eingangs genannten Art ist der US-A 5 318 016 zu entnehmen, wobei jedoch die Öffnung für eine Verbindung mit einem Aerosolabgabegerät und eine Öffnung mit einem Mundstück zum Abziehen des Aerosols an einer gemeinsamen Bodenfläche angeordnet sind, wodurch im Inneren der Kammer des Inhalationshilfsgerätes eine Umkehr der Bewegungsrichtung des Aerosols erforderlich wird.

Weiters ist aus der GB-A 2 110 543 ein Inhalationshilfsgerät bekanntgeworden, wobei eine kegelstumpfförmige Kammer mit veränderbarem Volumen durch eine Vielzahl von ineinanderschiebbaren, ringförmigen Elementen gebildet wird, wobei die Ringe durch jeweils geringeren Durchmesser zwischen aufeinanderfolgenden Ringen ineinander geschoben werden können.

Ein weiteres Problem bei bekannten Inhalationshilfsgeräten besteht darin, daß jeder Anbieter von Dosier-Aerosolen üblicherweise seinen eigenen Spacer anbietet, der nur mit den an diesen angepaßten, eigenen Aerosolabgabegeräten und Dosier-Aerosolen verwendbar ist. Damit wird es dem Anwender nahezu unmöglich gemacht Produkte von einem anderen Hersteller in Verbindung mit dem vorhandenen Spacer zu verwenden.

Die vorliegende Erfindung zielt nun darauf ab, ausgehend von einem Inhalationshilfsgerät bzw. Spacer der eingangs genannten Art, dieses so zu gestalten, daß es bei einfacher Konstruktion für einen Transport in eine Stellung bzw. Position gebracht werden kann, in welcher es einen verringerten Platzbedarf aufweist, wobei die vorliegende Erfindung insbesondere darauf abzielt, das Inhalationshilfsgerät für unterschiedliche Einsatzzwecke und mit unterschiedlichen Ausbildungen von Aerosolabgabegeräten verwenden zu können und insbesondere eine geschützte Transportstellung zu erzielen. Zur Lösung dieser Aufgabe ist das erfindungagemäße Inhalationshilfsgerät im wesentlichen dadurch gekennzeichnet, daß die Kammer in einem Zustand mit maximalem Volumen in wesentlichen zylindrisch oder kegelförmig mit zwei die Öffnungen ausweisenden, an gegenüberliegenden Enden der Kammer angeordneten Bodenelementen ausgebildet ist, daß die Begrenzungswände in Längsrichtung der Kammer von einem faltenbalgartigen Material oder von ineinander schiebbaren ringförmigen Elementen gebildet sind und daß eine Verschluß- bzw. Verriegelungseinrichtung in mit der Kammer gekoppelter Position die Öffnungen der Kammer in der Stellung verringerten Volumens überdeckt bzw. abschließt. Dadurch, daß erfindungsgemäß die Kammer in einem Zustand mit maximalem Volumen im wesentlichen zylindrisch oder kegelförmig mit zwei die Öffnungen aufweisenden, an gegenüberliegenden Enden der Kammer angeordneten Bodenelementen ausgebildet ist und daß die Begrenzungswände in Längsrichtung der Kammer von einem faltenbalgartigen Material oder von ineinander schiebbaren ringförmigen Elementen gebildet sind, läßt sich einfach ein eine im wesentlichen rohr- oder kugelförmige Kammer aufweisendes Inhalationshilfsgerät schaffen, wobei durch Verwendung eines faltenbalgartigen Materials oder ineinander schiebbarer, ringförmiger Elemente die Kammer in einem Zustand vergrößerten Volumens eine entsprechend hohe Eigenstabilität aufweist und aufgrund der konstruktionsbedingten Flexibilität des faltenbalgartigen Materials oder der ineinander schiebbaren Ringe nach der Benutzung auf ein wesentlich verringertes Volumen zusammengeschoben und derart einfach verwahrt werden kann. Durch Anordnung der Öffnungen in den die im wesentlichen rohrförmige oder kegelförmige Kammer begrenzenden, an gegenüberliegenden Ende der Kammer liegenden Bodenelementen wird für die Vorbereitung des Inhalationsvorganges das jeweils gewünschte, maximale Volumen der Kammer des Inhalationshilfsgerätes zur Verfügung gestellt und somit eine sichere und zuverlässige Vorverdampfung der den Wirkstoff umgebenden Primärtröpfchen ohne Strömungsumkehr erzielt. Durch eine erfindungsgemäße Verschluß- bzw. Verriegelungseinrichtung läßt sich weiters eine entsprechende Schutzfunktion während des Nichtgebrauches des Inhalationshilfsgerätes erzielen. In dem Zustand minimalen Volumens der Kammer ist erfindungsgemäß vorgesehen, durch die Verschluß- bzw. Verriegelungseinrichtung in mit der Kammer gekoppelter Position die Öffnungen der Kammer zu überdecken bzw. abzuschließen, wodurch sich zuverlässig ein Eindringen von Fremdteilchen während des Transportes in das Innere der Kammer vermeiden und somit ein hygienischer Transport erzielen läßt.

Für eine einfache Anpassung des Inhalationshilfsgerätes an die Verwendung mit unterschiedlichen Aerosolabgabegeräten bzw. zum Anschluß an unterschiedliche Abgabeöffnungen derselben ist einerseits vorgesehen, daß die Öffnung für die Verbindung mit dem Aerosolabgabegerät mit veränderbarem Querschnitt ausgebildet ist. Durch den veränderbaren Querschnitt läßt sich eine einfache Anpassung an unterschiedliche Aerosolabgabegeräte bei kleinstmöglichem Volumen für den Transport erzielen, so daß unabhängig von dem Hersteller bzw. dem verwendeten Produkt des Dosier-Aerosols mit einer einheitlichen Form des Inhalationshilfsgerätes das Auslangen gefunden werden kann. Falls lediglich geringfügige Unterschiede, beispielsweise im Durchmesser der Abgabeöffnungen von Aerosolabgabegeräten, berücksichtigt werden müssen, kann alternativ oder zusätzlich vorgesehen sein, daß die Öffnung für die Verbindung mit dem Aerosolabgabegerät in einer Begrenzungswand der Kammer aus elastischem Material ausgebildet ist. Es läßt sich somit auch der Herstellungsvorgang des erfindungsgemäßen Inhalationshilfsgerätes beträchtlich vereinfachen, da mit einheitlichen und einfachen Elementen das Auslangen gefunden werden kann und somit große Stückzahlen realisierbar sind. Da weiters die Kammer des Inhalationshilfsgerätes mit veränderbarem Volumen ausgebildet ist, wird es möglich, daß das Inhalationshilfsgerät für den Transport bzw. die Aufbewahrung in eine Stellung gebracht wird, in welcher es einen reduzierten Platzbedarf bei geschütztem Transport aufweist. Derart kann das erfindungsgemäß mit unterschiedlichen Aerosolabgabegeräten verwendbare Inhalationshilfsgerät ohne weiteres auch in kleineren Taschen aufbewahrt und mitgeführt werden, so daß das Hilfsgerät gemeinsam mit dem Aerosolabgabegerät ohne weiteres jederzeit verfügbar ist. Weiters kann aufgrund der Tatsache, daß die Kammer des Inhalationshilfsgerätes mit veränderbarem Volumen ausgebildet ist, das für eine Vorbereitung einer Inhalation zur Verfügung stehende Innenvolumen der Kammer des Inhalationshilfsgerätes an unterschiedliche Erfordernisse, beispielsweise an unterschiedliche Mengen des zu inhalierenden Medikamentes oder beispielsweise in Abhängigkeit davon, ob das Gerät von einem Kind oder einem Erwachsenen mit unterschiedlichem Atemvolumen verwendet werden soll, angepaßt werden.

Neben einer entsprechenden Verriegelung kann für eine weitere Verbesserung des Schutzes des Inhalationshilfsgerätes beim Transport darüberhinaus vorgesehen sein, daß die Verschluß- bzw. Verriegelungseinrichtung als kappenartiges Element ausgebildet ist, welches die Kammer in der Stellung verringerten Volumens wenigstens teilweise umgibt. Durch ein derartiges kappenartiges Element, welches die Kammer des Inhalationshilfsgerätes wenigstens teilweise umgibt, läßt sich eine einfache Verriegelung und eine verbesserte Schutzfunktion während des Nichtgebrauches des Inhalationshilfsgerätes erzielen.

Für eine konstruktiv besonders einfache Lösung zur Anpassung an unterschiedlichste Austrittsöffnungen der Aerosolabgabegeräte wird erfindungsgemäß bevorzugt vorgeschlagen, daß die Öffnung für die Verbindung mit einem Aerosolabgabegerät von einem starren, sich zum Inneren der Kammer trichterförmig verjüngenden Ansatzelement begrenzt ist, wodurch sich ein entsprechend dichter Anschluß bei Verwendung unterschiedlicher Aerosolabgabegeräte leicht erzielen läßt.

Unabhängig von der Form der Kammer des Inhalationshilfsgerätes ist für eine ordnungsgemäße Aufbereitung des Wirkstoffes die Anordnung der Öffnungen der Kammer bevorzugt so getroffen, daß die die Öffnungen der Kammer aufweisenden Bodenelemente in einer Stellung der Kammer maximalen Volumens einen maximalen Abstand voneinander aufweisen.

Für eine einfache Reinigung des Inneren der Kammer ist darüberhinaus bevorzugt vorgesehen, daß wenigstens eines der eine der Öffnungen aufweisenden Bodenelemente abnehmbar ausgebildet ist.

Wie oben bereits ausführlich erläutert, liegt ein wesentlicher Vorteil des erfindungsgemäßen Inhalationshilfsgerätes darin, daß es in einer zusammengeschobenen Stellung bzw. einem Zustand verringerten Volumens einen reduzierten Platzbedarf aufweist und somit leicht und einfach transportiert werden kann. Um für diesen Zweck, insbesondere bei Verwendung von vorgespannten Federelementen, ringförmigen Elementen oder entsprechend elastischem Material, eine sichere Einnahme und Aufrechterhaltung der Position verringerten Volumens der Kammer sicherzustellen, ist bevorzugt die Ausbildung so getroffen, daß die Kammer in einer Stellung verringerten Volumens mit der Verschluß- bzw. Verriegelungseinrichtung arretierbar ist.

Für eine besonders einfache Ausbildung, bei welcher mit möglichst wenigen Bauteilen das Auslangen gefunden werden kann, ist erfindungsgemäß bevorzugt vorgesehen, daß die die Kammer begrenzenden Bodenelemente mit Verriegelungseinrichtungen, insbesondere zueinander komplementären Vorsprüngen und Ausnehmungen oder Rastelementen, ausgebildet sind, welche in der Stellung verringerten Volumens der Kammer miteinander verriegelbar oder verrastbar sind und vorzugsweise die verschiebbaren oder faltenbalgartigen Begrenzungselemente im wesentlichen vollständig umschließen. Derartige Verriegelungselemente, wie beispielsweise Vorsprünge und Ausnehmungen oder Rastelemente, können leicht an den Bodenelementen vorgesehen oder angeformt werden. Bei im wesentlichen vollständigem Umschließen in geschlossenem Zustand läßt sich darüberhinaus eine entsprechend geschützte Transportstellung erzielen.

Gemäß einer weiteren bevorzugten Ausführungsform ist die Ausbildung so getroffen, daß insbesondere variable Begrenzungselemente für die Begrenzung bzw. Einstellung des Volumens der Kammer vorgesehen sind. Derartige Begrenzungselemente für die Ausfahrbewegung der Kammer bzw. zur Begrenzung oder Einstellung des Volumens derselben dienen zum einen dazu, eine Überbeanspruchung des den Mantel der Kammer bildenden Materials im Sinne einer übermäßigen Dehnung mit Sicherheit zu vermeiden. Bei vorsehen von variablen bzw. variabel einstellbaren Begrenzungselementen kann andererseits darüberhinaus auf einfache Weise das für die Vorbereitung des Inhalationsvorganges zur Verfügung gestellte Innenvolumen der Kammer in entsprechend weiten Grenzen angepaßt werden, so daß beispielsweise für Kinder ein entsprechend geringeres Volumen und für Erwachsene ein entsprechend größeres Volumen eingestellt werden kann.

Für die Erzielung eines veränderbaren Volumens der Kammer des erfindungsgemäßen Inhalationshilfsgerätes ist gemäß einer bevorzugten Ausführungsform die Ausbildung so getroffen, daß die Begrenzungswände der Kammer in Längsrichtung derselben aus einem faltbaren Material gebildet sind, welches als ein in der Stellung verringerten Volumens der Kammer vorgespanntes Federelement ausgebildet ist oder dieses enthält. Es sind eine Mehrzahl von dichten und auch gegenüber den eingesetzten Wirkstoffen inerten bzw. therapeutisch unbedenklichen Materialien einsetzbar, wobei beispielsweise bei Ausbildung eines im wesentlichen rohrförmigen Elementes aus faltbarem Material an dessen Umfang ein vorgespanntes Federelement enthalten ist. Derart kann in einfacher Weise die Kammer des Inhalationshilfsgerätes von einem Zustand mit maximalem Volumen, welches durch die Maße des faltbaren Materials bestimmt wird, durch einfaches Zusammendrücken der Feder auf ein entsprechend verringertes Volumen reduziert werden, wodurch sich ein einen einfachen Transport und eine einfache Lagerung ermöglichendes Gerät ergibt. Alternativ kann naturgemäß auch ein Material verwendet werden, welches inhärent die erforderliche Eigenspannung besitzt.

Anstelle eines faltenbalgartigen Materials kann, wie dies oben bereits angedeutet wurde, auch eine Vielzahl von ineinander schiebbaren, ringförmigen Elementen verwendet werden, um eine Kammer des Inhalationshilfsgerätes mit variablem Volumen zu erzielen. Für eine konstruktiv besonders einfache Ausführung wird erfindungsgemäß bevorzugt vorgeschlagen, daß die ineinander schiebbaren ringförmigen Elemente der Begrenzungswände der Kammer in Längsrichtung aus konisch verlaufenden Ringen gebildet sind. Durch Verwendung von derartig konisch verlaufenden Ringen läßt sich in einfacher weise auch eine entsprechende Abdichtung in geöffnetem Zustand der Kammer erzielen.

Für eine Verbesserung des Strömungsverhaltens im Inneren der Kammer des Inhalationshilfsgerätes ist darüberhinaus bevorzugt vorgesehen, daß vor der Austrittsöffnung zum Abziehen des Aerosols im Inneren der Kammer Leiteinrichtungen vorgesehen sind. Durch derartige Leiteinrichtungen läßt sich auch sicherstellen, daß die eingesetzte Wirkstoffmenge möglichst vollständig zu Behandlungszwecken umgesetzt wird.

Um eine entsprechend große Verweilzeit der Medikamentenpartikel im Inneren der Kammer des Inhalationshilfsgerätes sicherzustellen, ist, wie eingangs ausführlich erläutert, ein entsprechend großes Volumen der Kammer erforderlich. Erfindungsgemäß ist hiebei vorgesehen, daß die Kammer ein Volumen von etwa 150 ml bis 1 000 ml, vorzugsweise 200 ml bis 500 ml, aufweist.

Für eine Vereinfachung der Handhabung des Inhalationshilfsgerätes gemeinsam mit einem Aerosolabgabegerät und insbesondere dazu, um einen Inhalationsvorgang vorbereiten zu können, ohne unmittelbar bei Abgabe eines Sprühstoßes aus dem Aerosolabgabegerät bereits das Mundstück des Inhalationshilfsgerätes in den Mund nehmen zu müssen, ist darüberhinaus erfindungsgemäß bevorzugt vorgesehen, daß in der das Mundstück aufweisenden Öffnung ein ventil angeordnet ist. Ein derartiges Ventil im Bereich des Mundstückes ermöglicht eine entsprechend einfache Vorbereitung eines Inhalationsvorganges, wobei nach Abgabe eines Sprühstoßes in das Innere der Kammer des Inhalationshilfsgerätes durch Einführen des Mundstückes in den Mund des Benutzers und gleichzeitiges Öffnen des Ventils ein Inhalationsvorgang ohne Schwierigkeiten durchgeführt werden kann.

Für ein einfaches Einführen des Mundstückes in den Mund des Benutzers und zur Erleichterung eines im wesentlichen dichten Umschließens desselben ist darüberhinaus die Ausbildung bevorzugt so getroffen, das Mundstück an seinem äußeren Ende ergonomisch, insbesondere oval, ausgebildet ist und einen Innendurchmesser von 10 - 20 mm, vorzugsweise etwa 15 mm, aufweist.

Die Erfindung wird nachfolgend anhand von in der Zeichnung dargestellten Ausführungsbeispielen näher erläutert. In dieser zeigen
Fig. 1 einen Längsschnitt durch eine erste Ausführungsform eines erfindungsgemäßes Inhalationshilfsgerätes mit einem schematisch angedeuteten Aerosolabgabegerät in einem Zustand maximalen Volumens der Kammer;
Fig. 2 eine schematische Darstellung des Inhalationshilfsgerätes in einem Zustand minimalen Volumens der Kammer und mit einer am Inhalationshilfsgerät angeordneten Verschluß- bzw. Verriegelungseinrichtung;
Fig. 3 eine schematische Ansicht in Richtung des Pfeiles III der Fig. 2 auf die Verschluß- bzw. Verriegelungseinrichtung;
Fig. 4 in einer zu Fig. 1 ähnlichen Darstellung einen Längsschnitt durch eine abgewandelte Ausführungsform eines erfindungsgemäßes Inhalationshilfsgerätes in einem Zustand maximalen Volumens der Kammer;
Fig. 5 das in Fig. 4 dargestellte Inhalationshilfsgerät in geschlossenem Zustand, d.h. in einem Zustand mit minimalem Volumen der Kammer;
Fig. 6 ein Bodenelement einer weiteren abgewandelten Ausführungsform eines erfindungsgemäßes Inhalationshilfsgeräces. wobei die Öffnung für die Verbindung mit dem Aerosolabgabegerät mit veränderbarem Querschnitt ausgebildet ist;
Fig. 7 in einer schematischen Darstellung ähnlich zu den Fig. 1 und 4 eine Seitenansicht einer weiteren abgewandelten Ausführungsform eines erfindungsgemäßes Inhalationshilfsgerätes;
Fig. 8 eine Teilansicht einer weiteren abgewandelten Ausführungsform eines erfindungsgemäßes Inhalationshilfsgerätes mit einer Leiteinrichtung im Bereich der Öffnung mit einem Mundstück zum Abziehen des Aerosols; und
Fig. 9 in einer zu Fig. 8 analogen Darstellung eine weitere abgewandelten Ausführungsform eines erfindungsgemäßes Inhalationshilfsgerätes mit einer abgewandelten Leiteinrichtung.

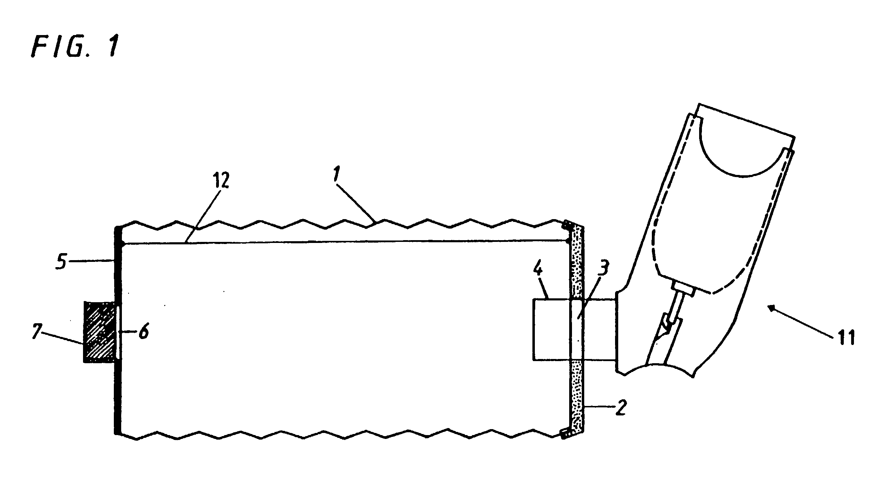

Das in Fig. 1 dargestellte Inhalationshilfsgerät umfaßt eine im wesentlichen zylindrische und zusammenschiebbare Zerstäubungskammer 1, die an einem Ende mit einem Boden 2 versehen ist, der eine Aufnahmeöffnung 3 für eine Austragsöffnung 4 eines schematisch angedeuteten Aerosolabgabegeräts 11 aufweist. Das andere Ende der Kammer 1 ist mit einem Boden 5 versehen, der eine Auslaßöffnung 6 aufweist, die in ein Mundstück 7 übergeht.

Das Mundstück 7 kann an dem äußeren Ende ergonomisch, z.B. oval, gestaltet werden. Der Innenraum des Mundstücks 7 hat einen Durchmesser von 10 - 20 mm, vorzugsweise 15 mm.

Der Boden 2 kann vorzugsweise aus einem weichen Material sein, das sich flexibel an die jeweiligen Formen der Austragsöffnung 4 des Aerosolabgabegerätes 11 anpaßt.

Die Zerstäubungskammer 1 kann wahlweise kugel-, kegel- oder birnenförmig und mit einem Volumen von 200 - 1000 ml gestaltet werden, wobei sie gemäß der dargestellten Ausführungsform vorzugsweise zylindrisch und mit einem Volumen von ca. 200 ml ausgebildet ist.

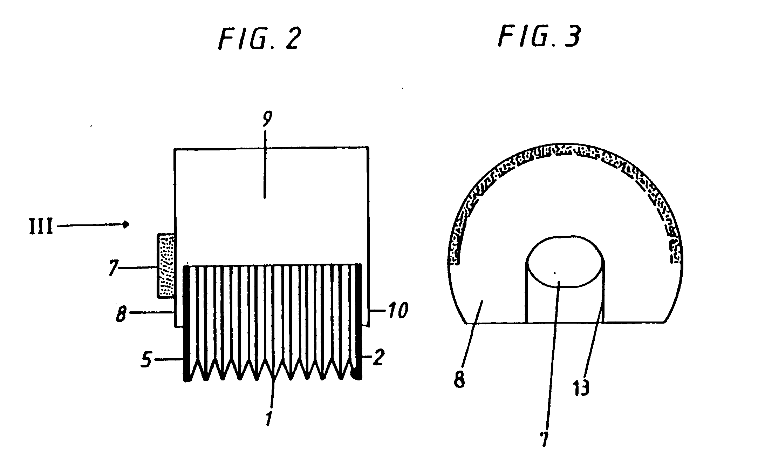

Das in Fig. 1 dargestellte Inhalationshilfsgerät weist in einem Zustand mit maximalem Volumen eine im wesentlichen zylindrische Kammer 1 auf, welche in Längsrichtung von einem faltenbalgartigen bzw. faltbaren Material begrenzt ist. Bei Verwendung eines faltenbalgartigen Materials läßt sich unmittelbar ein entsprechendes Zusammenschieben in die in Fig. 2 dargestellte Position und ein Entfalten aus dieser Position in die in Fig. 1 dargestellte Position erzielen. Alternativ kann ein faltbares Material verwendet werden, welches über seinen Umfang ein entsprechend vorgespanntes Federelement aufweist, so daß durch die Federkraft die in Fig. 1 dargestellte Stellung der Kammer selbsttätig einnehmbar ist. Um eine übennäßige Beanspruchung des Materials der Kammer 1 bzw. eine übermäßige Dehnung desselben zu vermeiden, ist darüberhinaus zwischen den Bodenelementen 2 und 5 ein Begrenzungselement 12 in Form eines Faden oder Seiles angeordnet, wobei die Länge dieses Begrenzungselementes 12 die maximal mögliche Erstreckung der Kammer 1 und somit das maximal einnehmbare Volumen definiert. Falls das Begrenzungselement beispielsweise durch eines der Bodenelemente 2 bzw. 5 hindurchgeführt ist, läßt sich in einfacher Weise durch Variaton der Länge des Begrenzungselementes 12 eine Variation des Innenvolumens der Kammer 1 in ausgefahrenem Zustand erzielen. Darüberhinaus ist der Boden 2 abnehmbar von dem Mantelmaterial der Kammer 1 ausgebildet, um eine einfache Reinigung des Inneren der Kammer 1 zu ermöglichen.

In der in Fig. 2 dargestellten Position befindet sich die Kammer 1 des Inhalationshilfsgerätes in einem Zustand verringerten Volumens, wobei eine Verschluß- bzw. Verriegelungseinrichtung als kappenartiges Element 9, welches in Fig. 3 in Seitenansicht dargestellt ist, ausgebildet ist, welches durch seine Begrenzungswände 8 bzw. 10 die Kammer wenigstens teilweise umgibt. Darüberhinaus wird die Öffnung im Bodenelement 2 durch die Seitenwand bzw. einen stegartigen Fortsatz 10 der Verschlußkappe 9 überdeckt, während das Mundstück 7 des Inhalationshilfsgerätes in einer entsprechenden Ausnehmung 13 in der Begrenzungswand 8 bzw. einem vorragenden Steg derselben des kappenförmigen Verschlußelementes 9 aufgenommen werden kann.

Das Mantelmaterial der Kammer 1, der Boden 5 und das Mundstück 7 der Ausbildung gemäß der Fig. 1 bis 3 können beispielsweise aus spritzgegossenem und somit einfach herzustellendem Material bestehen. Ebenso kann die Verschluß- bzw. Verriegelungseinrichtung 9 aus spritzgegossenem Material bestehen. Falls ein entsprechend ästhetisches Äußeres der gleichzeitig eine Abdeckung bzw. Schutzvorrichtung darstellenden Verschluß- bzw. Verriegelungseinrichtung 9 bzw. eine entsprechende gestalterische Ausbildung derselben erzielt werden soll, kann die Verschlußkappe 9 selbstverständlich auch aus therapeutisch unbedenklichem Metall oder Edelmetall hergestellt sein.

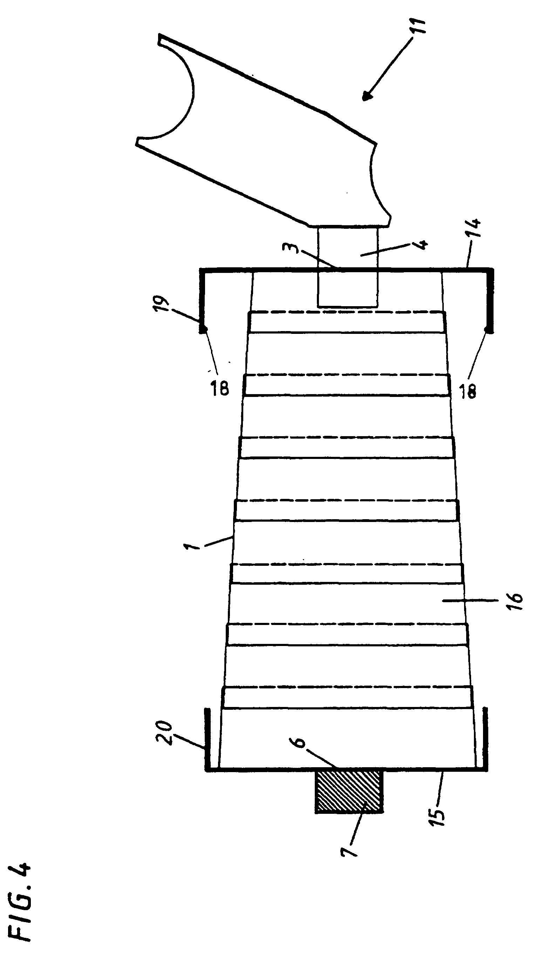

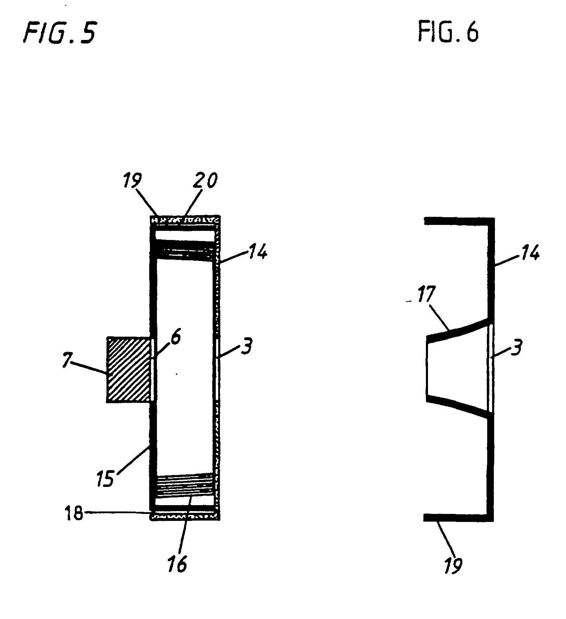

Bei der abgewandelten Ausführungsform gemäß den Fig. 4 und 5 sind die Bezugszeichen der vorangehenden Figuren für gleiche Beuteile beibehalten worden. Anstelle eines faltenbalgartigen Materials für die Begrenzungswände der Kammer 1 in Längsrichtung ist bei dieser Ausführungsform eine Vielzahl von konisch zulaufenden und ineinander schiebbaren, ringförmigen Elementen 16 vorgesehen. Auch bei dieser Ausführungsform ist an einem Boden 14 ein wiederum mit 11 bezeichnetes Aerosolabgabegerät an einer Öffnung 3 festlegbar, wobei am gegenüberliegenden Ende der aus ringförmigen Einzelelementen 16 gebildeten Zerstäubungskammer ein weiteres Bodenelement 15 vorgesehen ist, wobei wiederum eine Auslaßöffnung 6 und ein Mundstück 7 vorgesehen sind. Die Bodenelemente 14 und 15 bei der Ausbildung gemäß den Fig. 4 und 5 sind hiebei mit im wesentlichen rechtwinkelig von den Bodenelementen vorragenden Fortsätzen 19 und 20 ausgebildet, welche einander in der in Fig. 5 dargestellten, zusammengeschobenen Position überlappen, wobei zusätzlich an den Fortsätzen 19 schematisch angedeutete Rastnasen 18 ausgebildet sind, welche in geschlossenem Zustand eine sichere Verriegelung ergeben. Wie dies aus Fig. 5 ersichtlich ist, sind in geschlossenem Zustand die Ringe 16 entsprechend ineinander geschoben, sodaß sich ein stark verringertes Transportvolumen ergibt. Für den Fall, daß die Fortsätze 19 und 20 im wesentlichen ringförmig ausgebildet sind und sich über den gesamten Umfang bzw. Rand der Bodenelemente 14 und 15 erstreckt. ergibt sich in geschlossenem Zustand ein vollständiges Umschließen der ringförmigen Elemente 16 und derart ein entsprechend geschützter Transportzustand.

Im in Fig. 4 dargestellten, ausgefahrenen Zustand bzw. im Zustand maximalen Volumens ist die Kammer 1 sehr dicht, wobei Luft nicht durch die Einzelelemente 16 bzw. durch die Spalte zwischen den einzelnen Elementen aufgrund der konischen Ausbildung angesaugt werden kann. Durch Vorsehen einer relativ glatten Innenwand ist eine einfache Reinigung möglich. Weiters ergibt der leicht konische Verlauf der einzelnen ringförmigen Elemente 16 und somit der gesamten Kammer 1 ein entsprechend günstiges Strömungsprofil, da der Sprühstoß aus dem Aerosolabgabegerät 11 auch im wesentlichen konisch ist. Derart wird erzielt, daß der Aufprallwinkel einzelner Primärtröpfchen nicht mehr so steil ist wie bei einer im wesentlichen zylindrischen Form, sodaß bei der dargestellten, konischen Ausbildung weniger Medikamentenpartikel an der Innenwand der die Zerstäubungskammer 1 bildenden, ringförmigen Elemente 16 anhaften und somit ein entsprechend vollständigere Entnahme des eingesetzten Wirkstoffes erzielbar ist.

Für eine besonders einfache Anfertigung können hiebei sowohl die einzelnen Ringe 16 als auch die Bodenelemente 14 und 15 aus spritzgegossenem Material, beispielsweise Kunststoff hergestellt werden. Für ein entsprechend großes Volumen kann der Durchmesser der einzelnen Elemente 16 zwischen 2 und 10 cm, vorzugsweise 5 bis 6 cm, liegen, wobei für einen entsprechend geringen Platzbedarf in der Transportstellung die Höhe der einzelnen ringförmigen Elemente 16 sowie der vorragenden Fortsätze 19 und 20 der Bodenelemente 14 und 15 zwischen 1 und 5 cm, vorzugsweise 1,5 bis 2 cm, betragen kann.

Bei der in Fig. 6 gezeigten Darstellung einer abgewandelten Form eines Bodenelementes 14 ist vorgesehen, daß im Bereich der Öffnung 3 für die Verbindung mit einem nicht näher dargestellten Aerosolabgabegerät ein sich zum Inneren der Kammer 1 trichterförmig verjüngendes Ansatzelement 17 vorgesehen ist, um eine entsprechend leichte Anpassung an unterschiedliche Ausbildungen der Abgabeöffnungen von Aerosolabgabegeräten zu ermöglichen.

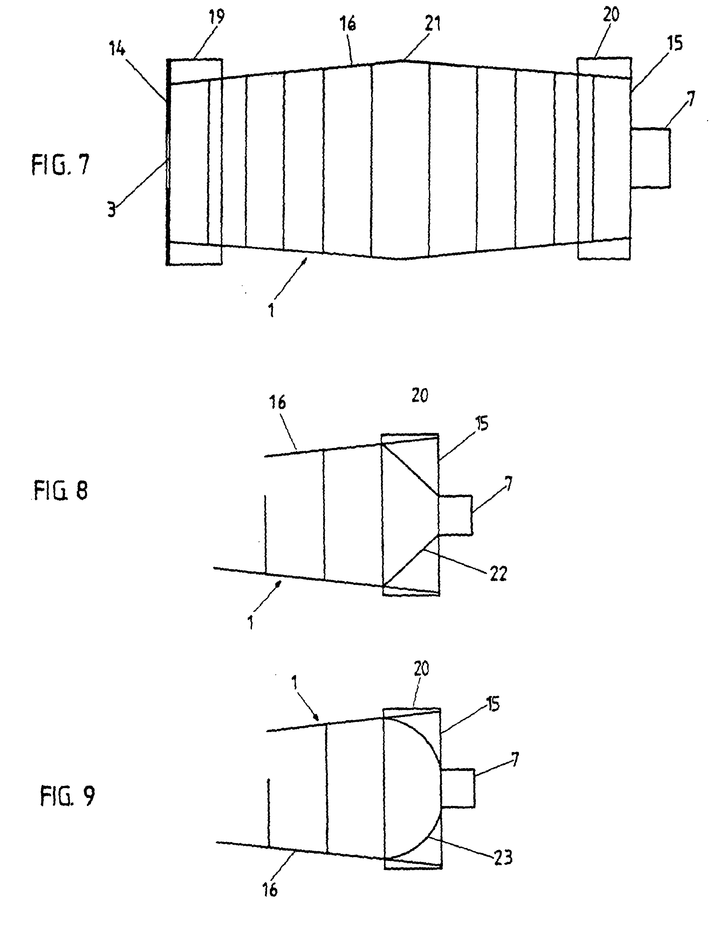

Bei einer abgewandelten Ausbildung gemäß Fig. 7 finden wiederum eine Mehrzahl von ineinander schiebbaren, ringförmigen Elementen Verwendung, wobei ein mittleres, ringförmiges Element 21 mit maximalem Durchmesser ausgebildet ist, während die zu beiden Seiten daran anschließenden, ringförmigen Elemente wiederum konisch zulaufend ausgebildet sind. Auch bei dieser Ausführungsform sind an den Bodenelementen 14 und 15 wiederum entsprechende Fortsätze bzw. beispielsweise umlaufende Ränder 19 und 20 vorgesehen, welche in geschlossenem Zustand des Inhalationshilfsgerätes eine entsprechende Verriegelung und Schutzfunktion ermöglichen. Die in Fig. 7 gezeigte Form der Kammer 1 mit zu beiden Enden konisch zulaufenden Begrenzungswänden ermöglicht hiebei eine weitere Anpassung und Optimierung des Strömungsverhaltens der Medikamentenpartikel bzw. allgemein des Wirkstoffes im Inneren der Zerstäubungskammer.

Für eine weitere Begünstigung des Strömungsverhaltens im Inneren der Kammer 1 ist bei der Ausbildung gemäß den Fig. 8 und 9 vorgesehen, daß im Bereich vor dem wiederum schematisch mit 7 bezeichneten Mundstück in der Kammer Leiteinrichtungen 22 bzw. 23 vorgesehen sind, wobei durch den optimierten Strömungsverlauf im Bereich unmittelbar vor der Austrittsöffnung mit dem Mundstück 7 ein entsprechend vollständigere Verwertung des eingesetzten Wirkstoffes erzielbar ist. Auch bei den Ausbildungen gemäß den Fig. 8 und 9 kann die Kammer 1 von ringförmigen Elementen 16 begrenzt sein, welche wiederum konisch zulaufend miteinander verbunden sind und einen entsprechend verringerten Platzbedarf in der Transportstellung ermöglichen.

## Patentansprüche

1. Inhalationshilfsgerät zur Verwendung in Verbindung mit Aerosolabgabegeräten (11), umfassend eine Kammer (1), welche mit einer Öffnung (3) für die Verbindung mit einem Aerosolabgabegerät (11) und mit einer Öffnung (6) mit einem Mundstück (7) zum Abziehen des Aerosols ausgebildet ist, wobei die Kammer (1) mit veränderbarem Volumen ausgebildet ist und wobei die Öffnung (3) für die Verbindung mit dem Aerosolabgabegerät (11) mit veränderbarem Querschnitt und/oder in einem Bodenelement (2) der Kammer (1) aus elastischem Material ausgebildet ist, **dadurch gekennzeichnet, daß** die Kammer (1) in einem Zustand mit maximalem Volumen im wesentlichen zylindrisch oder kegelförmig mit zwei die Öffnungen (3, 6) aufweisenden, an gegenüberliegenden Enden der Kammer angeordneten Bodenelementen (2, 5; 14, 15) ausgebildet ist, daß die Begrenzungswände in Längsrichtung der Kammer (1) von einem faltenbalgartigen Material oder von ineinander schiebbaren ringförmigen Elementen (16) gebildet sind und daß eine Verschluß- bzw. Verriegelungseinrichtung (9) in mit der Kammer (1) gekoppelter Position die Öffnungen (3, 6) der Kammer (1) in der Stellung verringerten Volumens überdeckt bzw. abschließt.

2. Inhalationshilfsgerät nach Anspruch 1, **dadurch gekennzeichnet, daß** die Verschluß- bzw. Verriegelungseinrichtung (9) als kappenartiges Element ausgebildet ist, welches die Kammer (1) in der Stellung verringerten Volumens wenigstens teilweise umgibt.

3. Inhalationshilfsgerät nach Anspruch 1 oder 2, **dadurch gekennzeichnet, daß** die Öffnung (3) für die Verbindung mit einem Aerosolabgabegerät (11) von einem starren, sich zum Inneren der Kammer (1) trichterförmig verjüngenden Ansatzelement (17) begrenzt ist.

4. Inhalationshilfsgerät nach einem der Ansprüche 1 bis 3, **dadurch gekennzeichnet, daß** die die Öffnungen (3, 6) der Kammer (1) aufweisenden Bodenelemente (2, 5; 14, 15) in einer Stellung der Kammer (1) maximalen Volumens einen maximalen Abstand voneinander aufweisen.

5. Inhalationshilfsgerät nach einem der Ansprüche 1 bis 4, **dadurch gekennzeichnet, daß** wenigstens eines der eine der Öffnungen (3, 6) aufweisenden Bodenelemente (2, 5; 14, 15) abnehmbar ausgebildet ist.

6. Inhalationshilfsgerät nach einem der Ansprüche 1 bis 5, **dadurch gekennzeichnet, daß** die Kammer (1) in einer Stellung verringerten Volumens mit der Verschluß- bzw. Verriegelungseinrichtung (9, 19, 20) arretierbar ist.

7. Inhalationshilfsgerät nach einem der Ansprüche 1 bis 6, **dadurch gekennzeichnet, daß** die die Kammer (1) begrenzenden Bodenelemente (19, 20) mit Verriegelungseinrichtungen (18), insbesondere zueinander komplementären Vorsprüngen und Ausnehmungen oder Rastelementen, ausgebildet sind, welche in der Stellung verringerten volumens der Kammer (1) miteinander verriegelbar oder verrastbar sind und vorzugsweise die verschiebbaren oder faltenbalgartigen Begrenzungselemente (16) im wesentlichen vollständig umschließen.

8. Inhalationshilfsgerät nach einem der Ansprüche 1 bis 7, **dadurch gekennzeichnet, daß** insbesondere variable Begrenzungselemente (12) für die Begrenzung bzw. Einstellung des Volumens der Kammer (1) vorgesehen sind.

9. Inhalationshilfsgerät nach einem der Ansprüche 1 bis 8, **dadurch gekennzeichnet, daß** die Begrenzungswände der Kammer (1) in Längsrichtung derselben aus einem faltbaren Material gebildet sind, welches als ein in der Stellung verringerten Volumens der Kammer (1) vorgespanntes Federelement ausgebildet ist oder dieses enthält.

10. Inhalationshilfsgerät nach einem der Ansprüche 1 bis 9, **dadurch gekennzeichnet, daß** die ineinanderschiebbaren ringförmigen Elemente (16) der Begrenzungswände der Kammer (1) in Längsrichtung aus konisch verlaufenden Ringen gebildet sind.

11. Inhalationshilfsgerät nach einem der Ansprüche 1 bis 10, **dadurch gekennzeichnet, daß** vor der Austrittsöffnung (6) zum Abziehen des Aerosols im Inneren der Kammer (1) Leiteinrichtungen (22, 23) vorgesehen sind.

12. Inhalationahilfsgerät nach einem der Ansprüche 1 bis 11, **dadurch gekennzeichnet, daß** die Kammer (1) ein Volumen von etwa 150 ml bis 1 000 ml, vorzugsweise 200 ml bis 500 ml, aufweist.

13. Inhalationshilfsgerät nach einem der Ansprüche 1 bis 12, **dadurch gekennzeichnet, daß** in der das Mundstück (7) aufweisenden Öffnung (6) ein Ventil angeordnet ist.

14. Inhalationshilfsgerät nach einem der Ansprüche 1 bis 13, **dadurch gekennzeichnet**, das Mundstück (7) an seinem äußeren Ende ergonomisch, insbesondere oval, ausgebildet ist und einen Innendurchmesser von 10 - 20 mm, vorzugsweise etwa 15 mm, aufweist.

## Claims

1. Inhalation aid for use in conjunction with aerosol delivery devices (11), comprising a chamber (1) constructed with an aperture (3) for connection to an aerosol delivery device (11) and with an aperture (6) with a mouthpiece (7) for drawing off the aerosol, the chamber (1) being constructed with variable volume and the aperture (3) for connection to the aerosol delivery device (11) being constructed with variable cross-section and/or in a base element (2) of the chamber (1) made of resilient material, **characterised in that** in a state with maximum volume the chamber (1) is constructed to be substantially cylindrical or conical with two base elements (2, 5; 14, 15) arranged at opposing ends of the chamber and comprising the apertures (3, 6), **in that** in the longitudinal direction of the chamber (1) the limiting walls are formed by a bellows-like material or by annular elements (16) which can be telescoped and **in that** in the position coupled to the chamber (1) a closing or locking device (9) covers or occludes the apertures (3,6) of the chamber (1) in the position of reduced volume.

2. Inhalation aid according to claim 1, **characterised in that** the closing or locking device (9) is constructed as a cap-like element at least partially surrounding the chamber (1) in the position of reduced volume.

3. Inhalation aid according to claim 1 or 2, **characterised in that** the aperture (3) for connection to an aerosol delivery device (11) is limited by a rigid shoulder (17) tapering in a funnel-shaped manner toward the interior of the chamber (1).

4. Inhalation aid according to any of claims 1 to 3, **characterised in that** the base elements (2, 5; 14, 15) comprising the apertures (3, 6) of the chamber (1) have a maximum distance from one another in a position of maximum volume of the chamber (1).

5. Inhalation aid according to any of claims 1 to 4, **characterised in that** at least one of the base elements (2, 5; 14, 15) comprising one of the apertures (3, 6) is constructed to be removable.

6. Inhalation aid according to any of claims 1 to 5, **characterised in that** the chamber (1) can be arrested by the closing or locking device (9, 19, 20) in a position of reduced volume.

7. Inhalation aid according to any of claims 1 to 6, **characterised in that** the base elements (19, 20) limiting the chamber (1) are constructed with locking devices (18), in particular projections and recesses or latching elements complementary to one another and lockable or latchable to one another in the position of reduced volume of the chamber (1) and preferably substantially completely enclosing the displaceable or bellows like limiting elements (16).

8. Inhalation aid according to any of claims 1 to 7, **characterised in that** in particular, variable limiting elements (12) are provided for limiting or adjusting the volume of the chamber (1).

9. Inhalation aid according to any of claims 1 to 8, **characterised in that** in their longitudinal direction the limiting walls of the chamber (1) are formed from a foldable material constructed as a spring element biased in the position of reduced volume of the chamber (1) or containing this spring element.

10. Inhalation aid according to any of claims 1 to 9, **characterised in that** the telescopic annular elements (16) of the limiting walls of the chamber (1) are formed in the longitudinal direction from conically extending rings.

11. Inhalation aid according to any of claims 1 to 10, **characterised in that** guide devices (22, 23) are provided in the interior of the chamber (1) upstream of the outlet aperture (6) for drawing off the aerosol.

12. Inhalation aid according to any of claims 1 to 11, **characterised in that** the chamber (1) has a volume of approximately 150 ml to 1,000 ml, preferably 200 ml to 500 ml.

13. Inhalation aid according to any of claims 1 to 12, **characterised in that** a valve is arranged in the aperture (6) comprising the mouthpiece (7).

14. Inhalation aid according to any of claims 1 to 13, **characterised in that** the mouthpiece (7) is ergonomic, in particular oval, in construction at its outer end and has an internal diameter of 10 to 20 mm, preferably approximately 15 mm.

## Revendications

1. Dispositif auxiliaire d'inhalation destiné à être utilisé en combinaison avec des dispositifs d'administration d'un aérosol (11), comprenant une chambre (1) comportant une ouverture (3) destinée à assurer la liaison avec le dispositif d'administration de l'aérosol (11) et une ouverture (6) avec un embout (7) pour l'aspiration de l'aérosol, la chambre (1) ayant un volume variable et l'ouverture (3) destinée à établir la liaison avec le dispositif d'administration de l'aérosol (11) ayant une section transversale variable et/ou étant agencée dans un élément de base (2) de la chambre (1), composé d'un matériau élastique, **caractérisé en ce que** la chambre (1) est constituée dans un état à volume maximal pour l'essentiel de façon cylindrique ou conique avec deux éléments de base (2, 5; 14, 15) comprenant les deux ouvertures (3, 6), agencées au niveau des deux extrémités opposées de la chambre, les parois de délimitation dans le sens longitudinal de la chambre (1) étant constituées par un matériau en forme de soufflet ou par des éléments annulaires pouvant être coulissés l'un dans l'autre (16), un dispositif de fermeture ou de verrouillage (9) recouvrant ou renfermant dans la position accouplée à la chambre (1) les ouvertures (3, 6) de la chambre (1) dans la position à volume réduit.

2. Dispositif auxiliaire d'inhalation selon la revendication 1, **caractérisé en ce que** le dispositif de fermeture ou de verrouillage (9) a la forme d'un élément de capot, entourant au moins partiellement la chambre (1) dans la position à volume réduit.

3. Dispositif auxiliaire d'inhalation selon les revendications 1 ou 2, **caractérisé en ce que** l'ouverture (3) destinée à la liaison avec un dispositif d'administration d'un aérosol (11) est limitée par un élément d'insertion (17) rigide, effilé en entonnoir vers l'intérieur de la chambre (1).

4. Dispositif auxiliaire d'inhalation selon l'une des revendications 1 à 3, **caractérisé en ce que** les éléments de base (2, 5; 14, 15) comportant les ouvertures (3, 6) de la chambre (1) sont espacés d'une distance maximale dans une position de la chambre (1) à volume maximal.

5. Dispositif auxiliaire d'inhalation selon l'une des revendications 1 à 4, **caractérisé en ce qu'**au moins un des éléments de base (2, 5; 14, 15) comportant les ouvertures (3, 6) est amovible.

6. Dispositif d'inhalation selon l'une des revendications 1 à 5, **caractérisé en ce que** la chambre (1) peut être bloquée par le dispositif de fermeture ou de verrouillage (9, 19, 20) dans une position à volume réduit.

7. Dispositif auxiliaire d'inhalation selon l'une des revendications 1 à 6, **caractérisé en ce que** les éléments de base (19, 20) délimitant la chambre (1) comportent des dispositifs de verrouillage (18), plus spécifiquement des saillies et des évidements ou des éléments d'encliquetage complémentaires, pouvant être verrouillés ou encliquetés dans la position à volume réduit de la chambre (1) et renfermant de préférence de façon pratiquement complète les éléments de délimitation (16) coulissants ou en forme de soufflet.

8. Dispositif auxiliaire d'inhalation selon l'une des revendications 1 à 7, **caractérisé en ce que** qu'il comporte plus spécifiquement des éléments de délimitation variables (12) pour la limitation ou le réglage du volume de la chambre (1).

9. Dispositif auxiliaire d'inhalation selon l'une des revendications 1 à 8, **caractérisé en ce que** les parois de délimitation de la chambre (1) sont constituées, dans le sens longitudinal de celle-ci, d'un matériau pliable, ayant dans la position à volume réduit de la chambre (1) la forme d'un élément élastique prétendu ou renfermant celui-ci.

10. Dispositif auxiliaire d'inhalation selon l'une des revendications 1 à 9, **caractérisé en ce que** les éléments annulaires pouvant être coulissés l'un dans l'autre (16) des parois de délimitation de la chambre (1) sont constitués dans le sens longitudinal par des anneaux à extension conique.

11. Dispositif auxiliaire d'inhalation selon l'une des revendications 1 à 10, **caractérisé en ce que** des dispositifs de guidage (22, 23) sont agencés devant l'ouverture de sortie (6) en vue de l'aspiration de l'aérosol à partir de l'intérieur de la chambre (1).

12. Dispositif auxiliaire d'inhalation selon l'une des revendications 1 à 11, **caractérisé en ce que** la chambre (1) a un volume compris entre environ 150 ml et 1 000 ml, de préférence entre 200 ml et 500 ml.

13. Dispositif auxiliaire d'inhalation selon l'une des revendications 1 à 12, **caractérisé en ce qu'**une soupape est agencée dans l'ouverture (6) comportant l'embout (7).

14. Dispositif auxiliaire d'inhalation selon l'une des revendications 1 à 13, **caractérisé en ce que** l'embout (7) a au niveau de son extrémité externe une forme ergonomique, plus spécifiquement ovale, et qu'il a un diamètre intérieur compris entre 10 et 20 mm, de préférence de l'ordre de 15 mm.
